Europäisches Patentamt

European Patent Office

Office européen des brevets

(11) Veröffentlichungsnummer: **0 067 146**
A1

## (12) EUROPÄISCHE PATENTANMELDUNG

(21) Anmeldenummer: **82890082.9**

(22) Anmeldetag: **01.06.82**

(51) Int. Cl.³: **B 01 D 3/00, C 12 F 1/00**

(30) Priorität: **05.06.81 AT 2528/81**

(43) Veröffentlichungstag der Anmeldung: **15.12.82**
**Patentblatt 82/50**

(84) Benannte Vertragsstaaten: **DE FR GB IT**

(71) Anmelder: **VEREINIGTE EDELSTAHLWERKE AKTIENGESELLSCHAFT (VEW), Elisabethstrasse 12, A-1010 Wien (AT)**

(72) Erfinder: **Schreier, Kurt, Dipl.-Ing., Tannengasse 14, A-2384 Breitenfurt (AT)**
Erfinder: **Bartsch, Karl, Dipl.-Ing., Fröbelgasse 60/3/17, A-1160 Wien (AT)**

(74) Vertreter: **Widtmann, Georg, Dr., Vereinigte Edelstahlwerke Aktiengesellschaft (VEW) Elisabethstrasse 12, A-1010 Wien (AT)**

(54) **Destillieranlage.**

(57) Destillieranlage zur Herstellung eines Destillates aus einem Wasser-Alkoholgemisch, bei der ein die zu destillierende Mischung aufnehmender Behälter einer Destillierapparatur (1) und ein in einem Kreislauf über eine Heizeinrichtung geführtes Fluid als Wärmeträger vorgesehen sind. Wobei die Heizeinrichtung eine Solaranlage (6) mit konzentrierenden Kollektoren ist und zwischen der Solaranlage (6) und der Destillierapparatur (1) ein Wärmespeicher (2), insbesondere Speicher für das, das Wasser-Alkoholgemisch erhitzende Fluid, zwischengeschaltet und eine weitere Heizeinrichtung (9) für das Wasser-Alkoholgemisch vorgesehen ist.

ENERGIEERZEUGUNG     DESTILLATION

## Destillieranlage

Die Erfindung bezieht sich auf eine Destillieranlage zur Herstellung eines Destillates aus einem Wasser-Alkoholgemisch, bei der ein die zu destillierende Mischung aufnehmender Behälter einer Destillierapparatur und ein in einem Kreislauf über eine Heizeinrichtung geführtes Fluid als Wärmeträger vorgesehen sind.

Für die Herstellung eines Destillates aus einem Wasser-Alkoholgemisch ist es bei obigen Anlagen notwendig, das Fluid auf etwa 90 °C bis 120 °C zu erhitzen. Bisher wurden dazu eigene Dampfkessel vorgesehen, oder wo dies möglich war, der Abdampf einer Niederdruck-Dampfturbine verwendet. Im ersteren Falle muß jedoch der Nachteil eines ungünstigen thermischen Wirkungsgrades in Kauf genommen werden. Dies ist dadurch bedingt, daß ein Prozeßdampf mit niederem Druck und relativ niederer Temperatur erforderlich ist, der in einem Dampfkessel eben nur mit einem ungünstigen thermischen Wirkungsgrad gewonnen werden kann.

Im zweiten Falle, bei Großanlagen, ergibt sich zwar durch die Kombination von Hochdruckkessel, Hochdruckturbine, Niederdruckturbine und die Verwendung des Abdampfes der letzteren als Prozeßdampf für die Destillation ein entsprechend hoher thermodynamischer Wirkungsgrad, doch ist diese Lösung auf Grund des komplizierten Energieflusses nur für Großanlagen geeignet. Dabei ergibt sich aber im Hinblick auf die Ausgangsprodukte zur Erzeugung des Wasser-Alkoholgemisches, wie zuckerhaltige oder stärkehaltige Pflanzen, Holzabfälle u. dgl., der Nachteil, daß große Mengen hievon erforderlich sind, um einen wirtschaftlichen Betrieb der Destillationsanlage zu ermöglichen, wodurch sich ein großer Einzugsbereich mit entsprechend langen Transportwegen für das Ausgangs- und Endprodukt ergibt.

Gerade die Erzeugung eines Destillates aus Biomasse, insbesondere zuckerhaltigen Pflanzen und Holz, gewinnt auf Grund der Verknappung und Verteuerung fossiler Energieträger steigende Bedeutung, wobei sich jedoch zeigt, daß deren Verwertung in umso größerem Ausmaß durchführbar ist, in je kleineren Einheiten gearbeitet werden kann, da sich dadurch der für die Zubringung der Ausgangsstoffe erforderliche Aufwand verringert.

Ziel der Erfindung ist es daher, eine Destillieranlage der eingangs erwähnten Art vorzuschlagen, die insbesondere auch für kleine Einheiten geeignet ist und die im Hinblick auf die Energieversorgung weitgehend unabhängig ist.

Erfindungsgemäß wird das dadurch erreicht, daß die Heizeinrichtung eine Solaranlage mit konzentrierenden Kollektoren ist, wobei zwischen der Solaranlage und der Destillierapparatur ein Wärmespeicher, insbesondere Speicher für das, das Wasser-Alkoholgemisch erhitzende Fluid, vorzugsweise ein Dampfspeicher, zwischengeschaltet ist und eine weitere Heizeinrichtung für das Wasser-Alkoholgemisch vorgesehen ist.

Dadurch ergibt sich auch der Vorteil eines einfachen Energieflusses. Außerdem erfolgt bei der erfindungsgemäßen Anlage die Aufheizung des die Alkohol-Wassermischung erhitzenden Fluids mit einer Einrichtung, die speziell für die Erzeugung von Dampf mit niedrigem Druck und niedriger Temperatur geeignet ist, und überdies eine kostenlose Primärenergie zur Erhitzung ausgenützt wird, wobei kurzzeitige Schwankungen des Energieangebotes durch den Speicher ausgeglichen werden können, wobei die Destillation auch bei längerem Ausfall einer Sonneneinstrahlung von genügender Intensität durch die Zusatzheizung aufrechterhalten werden kann. Die Zusatzheizung hat jedoch nicht nur den dort angeführten Vorteil, sondern ermöglicht es noch, die Solaranlage kleiner zu dimensionieren, da beim jeweiligen Beginn einer Destillation der Aufheizprozeß wesentlich kürzer durch zusätzlichen Betrieb der Zusatzheizung gehalten werden kann. Dieser Gesichtspunkt ist insbesondere auch deshalb von Be-

deutung, da üblicherweise Anlagen mit Arbeitsbeginn, somit am Morgen in Betrieb genommen werden und zu diesem Zeitpunkt die Sonneneinstrahlung noch geringer ist. Erst die Kombination der Solaranlage mit der Zusatzheizung erlaubt es, mit geringen Mitteln eine derartige Destillationsanlage zu betreiben. Der weiters vorgesehene Wärmespeicher dient jedoch lediglich zur Überbrückung von relativ kurzfristigen Unterbrechungen der Sonneneinstrahlung mit hoher Intensität, wobei bei längerfristigen Unterbrechungen die fehlende Wärme erneut durch Betrieb der Zusatzheizung beigesteuert wird.

Durch die erfindungsgemäße Destillieranlage wird es erstmals möglich, auf wirtschaftliche Art und Weise den Betrieb über lange Zeiten sicherzustellen, wobei dies von besonders hoher Bedeutung ist, da zur Erzeugung des Wasser-Alkoholgemisches aus Biomassen eine Vergärung stattfinden muß, die vergärten Substanzen in der Regel jedoch bei entsprechend schnell geführter Gärung nicht unbeschränkt lagerfähig sind und somit einer zeitmäßig gesicherten Verarbeitung zugeführt werden müssen.

Gemäß einem weiteren Merkmal der Erfindung ist der Wärmespeicher als Wärmetauscher zwischen dem Fluid der Solaranlage und einem weiteren, das Wasser-Alkoholgemisch erhitzenden Fluid, ausgebildet. Auf diese Art und Weise werden zwei getrennte Kreisläufe erhalten, womit für jeden Kreislauf das optimale Medium verwendet werden kann. Zum Beispiel kann für die Solaranlage ein Medium verwendet werden, das drucklos arbeitet, z.B. ein Paraffinöl, und für den weiteren Kreislauf Wasser, z.B. Sattdampf.

Gemäß einer bevorzugten Ausführungsform der erfindungsgemäßen Destillieranlage wird die weitere Heizeinrichtung mit Gas betrieben. Einerseits kann Gas relativ leicht gespeichert und transportiert werden, z.B. in Form von Flüssiggas, und anderseits kann Biogas verwendet werden, das durch anaerobe Gärung der bei der Destillation anfallenden Schlempe gewinnbar ist. Die Abhängigkeit von fossilen Primärenergieträgern kann auf diese Art und Weise entweder vollkommen vermieden werden bzw. auf ein geringstes Maß reduziert sein.

Ist im Wärmespeicher das das Wasser-Alkoholgemisch erwärmende Fluid als energiespeicherndes Medium vorgesehen, so kann als Fluid für die Solaranlage ein beliebiges Medium verwendet werden, gleichgültig ob gasförmig oder flüssig, wohingegen das das Wasser-Alkoholgemisch erwärmende Fluid so gewählt werden kann, daß im Speicher eine Phasenumwandlung stattfindet, wodurch ein Latentwärmespeicher auf besonders einfache Art und Weise erreichbar ist.

Wird ein thermisch isolierter Lagertank für das Fluid der Solaranlage vorgesehen, so kann bei längeren Betriebsunterbrechungen, z.B. über Nacht, auch diese Wärmemenge, die in dem Fluid vorhanden ist, gespeichert werden, wobei dann die Inbetriebnahme der gesamten Destillieranlage in den Morgenstunden besonders schnell vor sich gehen kann.

Die in üblicher Weise ausgebildete, einen Behälter zur Aufnahme des zu destillierenden Wasser-Alkoholgemisches aufweisende Destillierapparatur 1 mit einer Kolonne wird mit einem Fluid, Dampf, beheizt, der über eine mit einem als Speicher dienenden Röhrenwärmetauscher 2 verbundene Rohrleitung 3 zugeführt wird und der das Kondensat über die Rohrleitung 4 zum Speicher 2 zurückführt.

Der Röhrenwärmetauscher ist so ausgebildet, daß in den Rohren das Fluid der Solaranlage geleitet wird, wohingegen der Außenraum, welcher ein wesentlich größeres Volumen als das der Rohre aufweist, als Druckbehälter ausgebildet ist, sodaß er als Wärmespeicher dient. Die Rohrschlangen bzw. Rohrbündel des Röhrenwärmetauschers dienen zum Transport des Fluids der Mitteltemperatursolaranlage 6, mit welcher sie über die Leitungen 5 und 7 verbunden ist. Als Wärmeträgermedium kann ein Silikonöl, ein Paraffinöl od. dgl. dienen, das sich bei den Temperaturen max. 180 $^\circ$C noch nicht zersetzt.

Zwischen den Leitungen 3 und 4 ist eine Verbindungsrohrleitung 8 vorgesehen, in welcher eine weitere Heizeinrichtung 9 vorgesehen ist. Diese Heizeinrichtung besteht im wesentlichen aus einem Gasbrenner, welcher das Kondensat in einem Kessel zum Verdampfen bringt. In der Kolonne wird, wie durch den Pfeil 10 schematisch dargestellt, das Wasser-Alkoholgemisch oben aufgegeben, wohingegen unten bei 11 die Schlempe abgezogen wird. Das Kopfprodukt, der Rohsprit, wird bei 12 abgezogen.

Die Schlempe kann sodann einer anaeroben Gärung zur Gewinnung von Biogas unterzogen werden, das seinerseits zum Betrieb der weiteren Gasheizung Verwendung findet.

In der Leitung 5 ist ein thermisch isolierter Lagertank 5a vorgesehen, welcher für die Aufnahme des gesamten erhitzten Fluids der Solaranlage dienen kann. Der Betrieb der Destillieranlage erfolgt nun derart, daß entweder kontinuierlich das Wasser-Alkoholgemisch bei 10 eingetragen und die Schlempe kontinuierlich bei 11 abgezogen wird, wobei ebenfalls kontinuierlich der Rohsprit bei 12 anfällt. Die Energiezufuhr erfolgt über die Solaranlage 6, dessen Fluid seine Wärme an das im Wärmespeicher 2 angeordnete Wasser, das als Wärmeträger für die Destillieranlage dient, abgibt. Dieses wird verdampft und gelangt zur Destillieranlage, kondensiert dort und wird erneut in den Wärmetauscher rückgeführt. Ist nun ein Ausfall oder eine Leistungsreduktion der Mitteltemperatursolaranlage gegeben, so kann bei einem geringfügigen Ausfall der als Dampfspeicher dienende Wärmespeicher die Zeitspanne überbrücken. Ist der Ausfall von längerer Dauer, so muß die weitere Heizeinrichtung 9 zur Erhitzung des Dampfes verwendet werden. Wird die Anlage abgeschaltet und soll die Wärme im Fluid der Solaranlage auch gespeichert werden, so wird diese in dem thermisch isolierten Lagertank 5a gespeichert. Zur erneuten Inbetriebnahme wird dann das noch heiße Fluid der Solaranlage erneut in den Fluidkreislauf der Solaranlage eingepumpt, worauf die weitere Heizeinrichtung 9 in Betrieb genommen wird, sodaß gemeinsam durch die Sonneneinstrahlung und die weitere Heizeinrichtung eine schnelle Inbetriebnahme der Destillieranlage erneut gewährleistet ist.

0067146

- 7 -

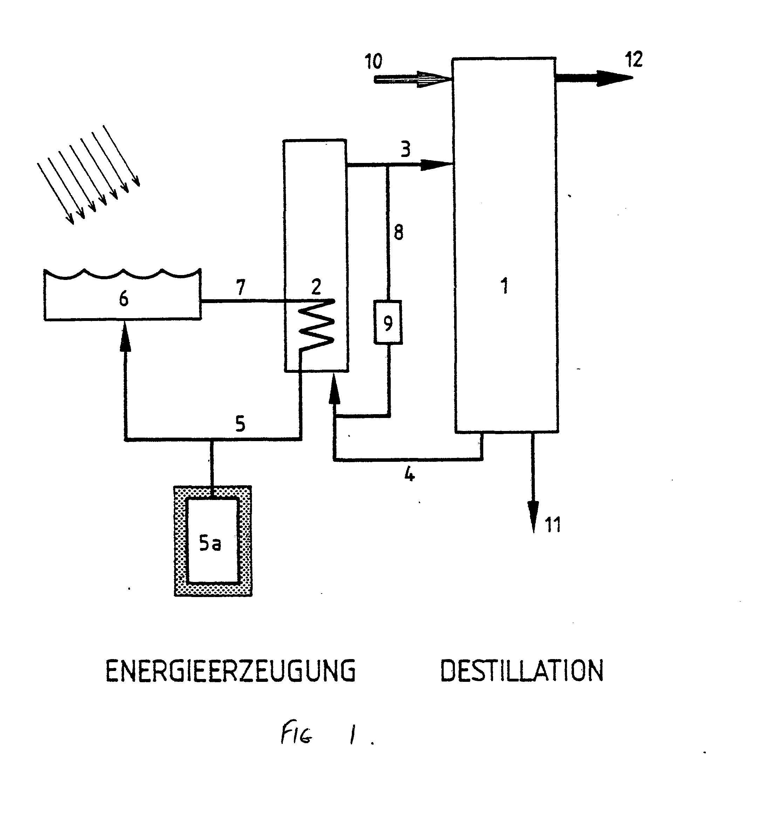

Patentansprüche :

1. Destillieranlage zur Herstellung eines Destillates aus einem Wasser-Alkoholgemisch, bei der ein die zu destillierende Mischung aufnehmender Behälter einer Destillierapparatur (1) und ein in einem Kreislauf über eine Heizeinrichtung geführtes Fluid als Wärmeträger vorgesehen sind, dadurch gekennzeichnet, daß die Heizeinrichtung eine Solaranlage (6) mit konzentrierenden Kollektoren ist, wobei zwischen der Solaranlage (6) und der Destillierapparatur (1) ein Wärmespeicher (2), insbesondere Speicher für das, das Wasser-Alkoholgemisch erhitzende Fluid zwischengeschaltet ist und eine weitere Heizeinrichtung (9) für das Wasser-Alkoholgemisch vorgesehen ist.

2. Destillieranlage nach Anspruch 1, dadurch gekennzeichnet, daß der Wärmespeicher (2) als Wärmetauscher zwischen dem Fluid der Solaranlage (6) und einem weiteren, das Wasser-Alkoholgemisch erhitzenden Fluid, ausgebildet ist.

3. Destillieranlage nach Anspruch 1 oder 2, dadurch gekennzeichnet, daß die weitere Heizeinrichtung (9) mit Gas betrieben ist.

4. Destillieranlage nach Anspruch 1, 2 oder 3, dadurch gekennzeichnet, daß im Wärmespeicher (2) das das Wasser-Alkoholgemisch erwärmende Fluid als energiespeicherndes Medium vorgesehen ist.

5. Destillieranlage nach einem der Ansprüche 1 bis 4, dadurch gekennzeichnet, daß ein thermisch isolierter Lagertank (5a) für das Fluid der Solaranlage vorgesehen ist.

ENERGIEERZEUGUNG          DESTILLATION

FIG 1.

# EUROPÄISCHER RECHERCHENBERICHT

EP 82 89 0082.9

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| Y | DE - A1 - 2 434 472 (F. BERGES) <br> * Anspruch 1; Fig. * <br> -- | 1 |
| Y | DE - A1 - 2 707 715 (H. FRITZEN) <br> * Fig. * <br> -- | 1 |
| Y | Patent Abstracts of Japan <br> Band 4, Nr. 173, 29. November 1980 <br> Seite 121C32 | 1 |
| A | & JP - A - 55 - 111890 <br> -- | 2 |
| P,Y | Patent Abstracts of Japan <br> Band 5, Nr. 204, 24. Dezember 1981 <br> Seite 75C85 | 1 |
| P | & JP - A - 56 - 124483 <br> -- | |
| A | DE - A1 - 2 744 022 (W. DERNEDDE) <br> * Fig. * <br> -- | 5 |
| A | US - A - 4 111 189 (E.S. DIZON) <br> * Fig. 1 * <br> -- | |
| A | US - A - 4 215 673 (S.S. COHEN) <br> * Fig. 1 * <br> -- | 1,5 |
| A | BE - A - 883 636 (J.-P. LABRIQUE) <br> * Fig. 1 * <br> -- | |

./..

**KLASSIFIKATION DER ANMELDUNG (Int. Cl.³)**

B 01 D 3/00

C 12 F 1/00

**RECHERCHIERTE SACHGEBIETE (Int. Cl.³)**

B 01 D 3/00

C 02 F 1/14

C 12 F 1/00

C 12 F 1/02

F 24 J 3/02

**KATEGORIE DER GENANNTEN DOKUMENTE**

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

Der vorliegende Recherchenbericht wurde für alle Patentansprüche erstellt.

| Recherchenort | Abschlußdatum der Recherche | Prüfer |
|---|---|---|
| Berlin | 23-07-1982 | KÜHN |

EPA form 1503.1  06.78

**Europäisches Patentamt**

**EUROPÄISCHER RECHERCHENBERICHT**

0067146

Nummer der Anmeldung

EP 82 89 0082.9
– Seite 2 –

| | EINSCHLÄGIGE DOKUMENTE | | KLASSIFIKATION DER ANMELDUNG (Int. Cl.³) |
|---|---|---|---|
| Kategorie | Kennzeichnung des Dokuments mit Angabe, soweit erforderlich, der maßgeblichen Teile | betrifft Anspruch | |
| A | Patent Abstracts of Japan<br><br>Band 4, Nr. 89, 25. Juni 1980<br><br>Seite 112C16<br><br>  &  JP – A – 55 – 54078<br><br>––––– | | RECHERCHIERTE SACHGEBIETE (Int. Cl.³) |